# EUROPÄISCHE PATENTSCHRIFT

(11) **EP 1 228 706 B1**
(45) Veröffentlichungstag und Bekanntmachung des Hinweises auf die Patenterteilung: **04.05.2005**
(21) Anmeldenummer: 01121958.1
(22) Anmeldetag: 12.09.2001
(51) Int. Cl.: A23L 1/305, A61K 31/195, A61K 31/40, A61K 31/44, A61K 31/51, A61K 33/34

(54) **Präparat zum Bindegewebsaufbau und zur Konditionierung bei hoher körperlicher Beanspruchung**
Composition for enhancing the forming of connective tissue and for conditioning the body for high physical performance
Compositions pour la formation de tissu conjonctif et à l'occasion d'une sollicitation corporelle elevée

(30) Priorität: 24.01.2001 DE 20101225 U
(43) Veröffentlichungstag der Anmeldung: 07.08.2002
(73) Patentinhaber: Sportmedizin Team Vertriebs GmbH, 9442 Berneck (CH)
(72) Erfinder: Apfelbaum, Udo, 9442 Berneck (CH); Prof. Weiss, Michael, Warburger Str.100, 33098 Paderborn (DE)
(74) Vertreter: Appelt, Christian W.

(56) Entgegenhaltungen:
- EP-A- 0 705 542
- EP-A- 0 712 583
- EP-A- 0 873 754
- WO-A-94/01006
- WO-A-99/01044
- DE-A- 4 139 639
- US-A- 5 026 721
- US-A- 5 310 768
- US-A- 5 480 865

## Beschreibung

Die Erfindung bezieht sich auf ein Präparat für den Bindegewebsaufbau und zur Konditionierung des Körpers bei hoher körperlicher Beanspruchung, und zwar speziell zur Unterstützung, zur Erhaltung und zum Aufbau von Binde- und Stützgewebe bei erhöhtem Aminosäurebedarf, zur Erhöhung der mechanischen und stoffwechselmäßigen Beanspruchbarkeit in Folge sportlichen Trainings oder schwerer körperlicher Arbeit sowie bei reduzierter Nährstoffzufuhr bei diätetischen Maßnahmen wie z. B. Gewichtsreduktion. Das Präparat dient auch zur Unterstützung des Heilungsprozesses an Sehnen und Bändern nach Verletzungen und zur Stärkung bei Trainingsprozessen. Ferner soll das Präparat die Zufuhr der Aminosäuren und Begleitstoffe sichern, die zur Erhaltung und Erneuerung der elastischen Gewebeanteile in den Gelenkknorpeln und in Haut und Unterhaut notwendig sind. Die Unterhaut bleibt dadurch straff und die Haut elastisch.

Das Präparat wird oral eingenommen, zweckmäßigerweise zu oder nach kohlehydratreicher Kost.

Das wichtigste Protein des Bindegewebes ist Kollagen, das schätzungsweise etwa ein Drittel der gesamten Proteinmasse des Menschen ausmacht. Der Grundbaustein aller Kollagenformen ist eine starre Helix aus drei Ketten, nämlich eine Tripelhelix aus Aminosäuren. Bei hoher körperlicher Beanspruchung, insbesondere bei sportlicher Belastung, beispielsweise bei Wettkämpfen, werden die Bänder und Sehnen und das sonstige Bindegewebe sehr stark in Anspruch genommen. Sowohl zu deren Stärkung und zum Vorbeugen vor Abbau bei katabolen Zuständen, als auch für den Wiederaufbau nach Verletzungen, oder bei Elastizitätsverlust der Haut sollen Versorgungsgrundlagen geschaffen werden, wobei zu beachten ist, daß sich die Aminosäurenzusammensetzung des Kollagens erheblich von der der Muskulatur und anderer Organe unterscheidet.

Aus der WO 94/01006 sind Nahrungsergänzungsmittel zur Verbesserung des Befindens bekannt, die ein Aminosäurengemisch enthalten, wobei in Ausführungsbeispielen dieses Gemisch neben zahlreichen anderen Aminosäuren auch Glycin, Prolin, Glutamin, Arginin, Lysin und Serin im gegenseitigen Gewichtsverhältnis von ungefähr 6 : 4 : 3 : 2 : 1 : 1 enthält. Auch ein Gemisch von Mineralstoffen ist enthalten, worin auch Kupfer umfaßt sein kann. Ein gezielt auf den Kollagenaufbau ausgerichtetes Wirkungsspektrum ergibt sich bei den bekannten Gemischen aber nicht.

Durch die Erfindung soll also hierfür eine sinnvolle Substitution und Supplementation zusammengestellt werden. Dieses ist durch das erfindungsgemäße Präparat gegeben, das ein Aminosäuregemisch enthält, welches aus Glycin, Prolin, Glutamin, Arginin, Lysin und Serin im Gewichtsverhältnis 6 : 4 : 3 : 2 : 1 : 1 besteht, wobei das Präparat zusätzlich eine kleine Menge Kupfer in Form eines Salzes, eine kleine Menge Vitamin B6 in der Form von Pyridoxalphosphat und eine kleine Menge Vitamin B1 enthält. Für die an der Kollagensynthese und Quervernetzung beteiligten Enzyme und deren Aktivierung sind noch als Enzymbestandteile und für die Umsetzungsreaktionen Vitamine und Spurenelemente erforderlich. Generell sind dies Kupfer und aus der Vitamin B6-Reihe Pyridoxalphosphat. Die Beimischung von Kupfer ist sinnvoll, da es bei starker Belastung immer wieder zu Engpässen in der Kupferzufuhr kommt. Zur Unterstützung des Kollagenaufbaus ist also noch Kupfer, z. B. als Gluconat oder Orotat, enthalten. Mit Vitamin B6 sieht es ähnlich aus: Man geht von einem erhöhten Bedarf beim hochbeanspruchten Menschen aus, weshalb noch Vitamin B6 in Form von Pyridoxalphosphat enthalten ist. Das ebenfalls im Präparat enthaltene Vitamin B1 dient als Stoffwechselaktivator. Vorzugsweise gehört zum erfindungsgemäßen Präparat weiterhin noch Vitamin C, das jedoch gesondert konfektioniert und den übrigen Bestandteilen nicht beigemischt ist. Für den wirkungsvollen Gebrauch wird das Vitamin C um ca. 2 Stunden verzögert eingenommen, damit die im Stammpräparat enthaltenen Mineralstoffe nicht oxidiert/reduziert werden.

Das angegebene Gewichtsverhältnis der Aminosäuren hat natürlich gewisse Toleranzen, die durch die Grenzen der Wirksamkeit gegeben sind. Es handelt sich um eine Zusammenstellung, die den Aufbau von Pro- und Tropo-Kollagen unterstützt. Damit ist die Grundlage zum Aufbau für die verschiedenen Kollagene gegeben, die in den verschiedenen Typen von Bindegeweben vorkommen und somit für die Anpassung an steigenden sportliche Belastung vor allem in der Regenerationsphase zuzuführen sind, wobei das Präparat auch katabolen Zuständen in Phasen sehr hoher Beanspruchung vorbeugt und nicht zuletzt auch dem Wiederaufbau nach einer Verletzung dient. Es dient also zum Schutz und Neuaufbau von Bindegewebe unter besonderer Berücksichtigung der Sicherung der Aminosäurenzufuhr. Die absoluten Mengen müssen sich natürlich daran messen, was noch wirksam ist. Vorzugsweise beträgt die Dosismenge des Glycin mindestens 150 mg, noch besser mindestens 300 mg, womit die Gewichtsanteile der anderen Komponenten aufgrund des Gewichtsverhältnisses gegeben sind.

Beim spezifischen erfindungsgemäßen Gemisch hat Glycin den höchsten Mengenanteil, da diese Aminosäure die zentrale Position in der Pro-alpha-Kette Glycin einnimmt. Glutamin wird als Starteraminosäure für die Synthese fast überall benötigt und ist wegen des hohen Anteils von 11,8 g pro 100 g Kollagen in größeren Mengen nötig. Zwar ist es die im Blutplasma und im Muskel am höchsten konzentrierte Aminosäure, aber zahlreiche Systeme und Organe, so z. B. das Immunsystem und die Glutathion-Synthese, konkurrieren um das Glutamin-Angebot, so daß der Bedarf dieser Aminosäure unter und nach sportlicher Belastung enorm gesteigert ist.

Für die Quervernetzung der Kollagenfibrillen sind die wichtigen Schlüsselstellen die Aminosäuren Prolin und Lysin, außerdem fällt Arginin noch quantitativ mit 9,1 g/100 g Kollagen bedeutsam ins Gewicht. In gleichen Abteilen kommt auch Alanin vor, das aber vom arbeitenden Muskel in größeren Mengen produziert wird und sehr hohe Serumspiegel aufweist. Engpässe mit Alanin treten sicher erst auf, wenn das Glykogen völlig verbraucht ist und der Organismus gezwungen ist, in der Leber große Mengen Glukose selbst über den Alanin-Glukose-Zyklus zu produzieren. Serin kommt mit 3,3 g in 100 g Kollagen vor, und auch mit dieser glukoplastischen und unter Belastung semiessentiellen Aminosäure kann ein Engpaß auftreten.

Auf eine Erhöhung der sonst noch für die Kollagene bedeutsamen Aminosäuren Asparagin, Leucin und Isoleucin zur Korrektur der Verschiebungen im Aminosäurenprofil durch Sport und körperliche Belastung kann man gemäß Untersuchungen der Erfinder verzichten, da diesbezüglich ein aktivierter Intermediärstoffwechsel stattfindet, so daß es zwar zu geringfügigem Abfallen in den Serumspiegeln kommt, aber nicht zu Versorgungsengpässen.

Die Einnahme des Präparats zusammen mit oder nach Kohlehydraten mit hohem glykämischem Index ist empfehlenswert. Als Beispiel sei folgende Darreichungsform angegeben: Das Präparat hat die Form von Dragees, alternativ kommen auch andere feste Formen wie Tabletten oder Kapseln in Frage, von denen beispielsweise dreimal täglich eines eingenommen wird. In diesem Fall enthält ein Dragee:

| | | | |
|---|---|---|---|
| L-Glycin | 354 mg | L-Prolin | 236 mg |
| L-Glutaminsäure | 177 mg | L-Arginin | 118 mg |
| L-Lysin | 59 mg | L-Serin | 59 mg |
| Vit. B6 | 1,33 mg | Kupfer | 0,33 mg |
| Vit. B1 | 0,2 mg | | |

Ein ergänzendes, zwei Stunden nach dem beschriebenen Dragee einzunehmendes Zusatzdragee enthält 54,47 g Ascorbinsäure (Vitamin C).

## Patentansprüche

1. Präparat für den Bindegewebsaufbau und zur Konditionierung des Körpers bei hoher körperlicher Beanspruchung, das ein Aminosäuregemisch enthält, welches aus Glycin, Prolin, Glutamin, Arginin, Lysin und Serin im Gewichtsverhältnis 6 : 4 : 3 : 2 : 1 : 1 besteht, wobei das Präparat zusätzlich eine kleine Menge Kupfer in Form eines Salzes, eine kleine Menge Vitamin B6 in der Form von Pyridoxalphosphat und eine kleine Menge Vitamin B 1 enthält.

2. Präparat nach Anspruch 1, **dadurch gekennzeichnet, daß** es in Einheiten dosiert ist, die jeweils wenigstens 150 mg Glycin enthalten.

3. Präparat nach Anspruch 2, **dadurch gekennzeichnet, daß** die Einheiten jeweils wenigstens 300 mg Glycin enthalten.

4. Präparat nach einem der Ansprüche 1 bis 3, **dadurch gekennzeichnet, daß** das Glycin, Prolin, Glutamin, Arginin, Lysin und/oder Serin in Form von linksdrehendem (= L) L-Glycin, Prolin, L-Glutaminsäure, L-Arginin, L-Lysin bzw. L-Serin vorliegen.

5. Präparat nach einem der Ansprüche 1 bis 4, **dadurch gekennzeichnet, daß** das Kupfer, das Vitamin B6 und/oder das Vitamin B1 in einer Menge von 0,05 % bis 0,5 % des Anteils des Glycins enthalten sind.

6. Präparat nach einem der Ansprüche 1 bis 5, **dadurch gekennzeichnet, daß** es als Dragee konfektioniert ist.

7. Präparat nach einem der Ansprüche 1 bis 6, **dadurch gekennzeichnet, daß** es ergänzt ist durch getrennt konfektioniertes, zeitlich getrennt einzunehmendes Vitamin C.

## Claims

1. Preparation for building up connective tissue and for conditioning the body in case of high physical strain, which contains an amino acid mixture consisting of glycine, proline, glutamine, arginine, lysine and serine in the weight ratio of 6 : 4 : 3 : 2 : 1 : 1, wherein the preparation additionally contains a small amount of copper in the form of a salt, a small amount of vitamin B6 in the form of pyridoxalphosphate and a small amount of vitamin B1.

2. Preparation according to claim 1, **characterized in that** it is given in doses containing at least 150 mg glycine each.

3. Preparation according to claim 2, **characterized in that** the doses contain at least 300 mg glycine each.

4. Preparation according to any one of claims 1 to 3, **characterized in that** the glycine, proline, glutamine, arginine, lysine and/or serine are present in the form of left-turning (= L) L glycine, L proline, L glutamine acid, L arginine, L lysine and L serine, respectively.

5. Preparation according to any one of claims 1 to 4, **characterized in that** the copper, the vitamin B6 and/or the vitamin B1 are present in an amount of 0.05 % to 0.5 % of the amount of the glycine.

6. Preparation according to any one of claims 1 to 5, **characterized in that** it is confectioned as a dragee.

7. Preparation according to any one of claims 1 to 6, **characterized in that** it is supplemented by separately confectioned vitamin C which is to be taken with a delay.

## Revendications

1. Composition pour la formation de tissu conjonctif et pour conditionner le corps à une sollicitation corporelle élevée, qui comporte un mélange d'acides aminés composé de glycine, de praline, de glutamine, d'arginine, de lysine et de sérine dans les proportions pondérales 6 : 4 : 3 : 2 : 1 : 1, cette composition comprenant de plus une faible quantité de cuivre sous la forme d'un sel, une faible quantité de vitamine B6 sous la forme de pyridoxalphosphate et une faible quantité de vitamine B1.

2. Composition selon la revendication 1, **caractérisée en ce qu'**elle est dosée sous la forme d'unités contenant chacune au moins 150 mg de glycine.

3. Composition selon la revendication 2, **caractérisée en ce que** les unités contiennent chacune au moins 300 mg de glycine.

4. Composition selon une des revendications 1 à 3, **caractérisée en ce que** la glycine, la proline, la glutamine, l'arginine, la lysine et/ou la sérine sont présentes sous la forme lévogyre (= L) de L-glycine, L-proline, d'acide L-glutaminique, L-arginine, L-lysine et/ou L-sérine.

5. Composition selon une des revendications 1 à 4, **caractérisée en ce que** le cuivre, la vitamine B6 et/ou la vitamine B1 sont contenus dans la proportion de 0,05 % à 0,5 % de la glycine.

6. Composition selon une des revendications 1 à 5, **caractérisée en ce qu'**elle est confectionnée sous forme de dragées.

7. Composition selon une des revendications 1 à 6, **caractérisée en ce qu'**elle est complétée par de la vitamine C confectionnée séparément et à prendre séparément dans le temps.
